# EUROPEAN PATENT APPLICATION

(11) **EP 3 944 250 A1**
(43) Date of publication of application: **26.01.2022**
(21) Application number: 20187232.2
(22) Date of filing: 22.07.2020
(51) Int. Cl.: G16H 15/00, G16H 10/00

(54) **METHODS AND SYSTEMS FOR SEARCHING AN ECG DATABASE**

(71) Applicant: Koninklijke Philips N.V., 5656 AG Eindhoven (NL)
(72) Inventor: JIN, Kim, 5656 AE Eindhoven (NL); GE, Essien, 5656 AE Eindhoven (NL)
(74) Representative: Philips Intellectual Property & Standards

(57) **Abstract**

The invention provides a method for searching a database of ECG data. The method includes, obtaining a reference set of ECG data, wherein the reference set of ECG data comprises ECG data for a plurality of subjects comprising, for each of said subjects, data values for a plurality of characteristic ECG features, and grouping the reference set of ECG data into a plurality of subsets of ECG data, wherein each of the plurality of subsets of ECG data is associated with one of a plurality of characteristic ECG features. A search feature extraction algorithm is applied to the reference set of ECG data to extract at least one characteristic ECG feature as a search feature wherein a search feature is a characteristic ECG feature which enables ECG data of interest to be identified within the database of ECG data and a search criterion is generated based on the search feature. The database is then searched using the search criterion to obtain ECG data of interest.

## Description

### FIELD OF THE INVENTION

The invention relates to the field of data handling, and more specifically to the field of database searching.

### BACKGROUND OF THE INVENTION

Research toolsets can help physicians work more efficiently on clinical research. Diagnostic electrocardiogram (ECG) data is widely used for clinical diagnosis and screening and physicians require many different, advanced tools to help them work on ECG based research.

An ECG management system may be used to manage all ECG data within a given database and may include, or facilitate, a research platform and/or toolset on the in order to provide a convenient means of conducting ECG related research.

One of the most important features of an ECG research toolset is a search function adapted to find data matching a given criteria. The search function is also usually the first module to be used in a research workflow for many research topics, as preparing data is typically the first step before subsequent processing. Accordingly, the search function plays a significant role in the research workflow as the data found using the search function will form the basis of the remaining research.

There is therefore a need for a means to search for data within a database to fulfil a given research requirement.

### SUMMARY OF THE INVENTION

The invention is defined by the claims.

According to examples in accordance with an aspect of the invention, there is provided a computer implemented method for searching a database of ECG data, the method comprising:
obtaining a reference set of ECG data, wherein the reference set of ECG data comprises ECG data for a plurality of subjects comprising, for each of said subjects, data values for a plurality of characteristic ECG features;
grouping the reference set of ECG data into a plurality of subsets of ECG data, wherein each of the plurality of subsets of ECG data comprises data values for a respective one of the one of a plurality of characteristic ECG features;
applying a search feature extraction algorithm to the reference set of ECG data to extract at least one of said characteristic ECG features as a search feature wherein a search feature is a characteristic ECG feature which enables ECG data of interest to be identified within the database of ECG data;
generating a search criterion based on the search feature; and
searching the database using the search criterion to obtain ECG data of interest.

The method provides for a means of searching an ECG database based on characteristic ECG features of a reference set of ECG data using an automatically generated search criterion.

By applying a search feature extraction algorithm to the reference set of ECG data, the most relevant search features may be extracted from the reference set of ECG for generating the search criterion.

In this way, the search feature of an ECG system may be extended to automatically extracting and searching for similarities extracted from a reference set of ECG data.

In an embodiment, the search feature extraction algorithm comprises:
normalizing each of the plurality of subsets of ECG data to generate a plurality of normalized subsets of ECG data;
calculating a variance for each of the plurality of normalized subsets of ECG data;
defining one or more first threshold values;
comparing the variance of each of the plurality of normalized subsets of ECG data with one of the one or more first threshold value; and
extracting at least one feature as a search feature based on the comparison.

In this way, search features may be extracted from the reference set of ECG data based on a measure of similarity, i.e. the variance, across the data in the subsets of ECG data. For example, if a subset of data form the reference set of ECG has a low variance, the data making up said subset has a high similarity across the subset. By generating the search feature based on a subset of data having a high similarity, it is possible to identify trends in a limited set of ECG data that may be used to search for similar subjects in the larger ECG database.

In an embodiment, defining one or more first threshold values comprises defining a first threshold value for a plurality of the normalized subsets of ECG data, and wherein the variance of each of the plurality of subsets of ECG data relating to the same characteristic ECG features as the plurality of normalized subsets of ECG data are compared with said first threshold value.

In this way, a threshold value may be defined for each of the characteristic ECG features in the reference set of ECG data set thereby increasing the control over the extraction of search features from the reference set.

In an embodiment, defining one or more first threshold value comprises defining a first threshold value for each of the plurality of subsets of ECG data, and wherein the variance of each of the plurality of subsets of ECG data are compared with the first threshold value defined for that subset of ECG data.

In this way, a global threshold may be defined for the entire reference set of ECG data.

In an embodiment, normalizing each of the plurality of subsets of ECG data comprises:
normalizing the subset of ECG data based on a maximum and minimum value of the subset of ECG data;
normalizing the subset of ECG data based on a maximum and minimum value of the database of ECG data associated with the same characteristic ECG feature as the plurality of subsets of ECG data; or
normalizing the subset of ECG data based on a known maximum and minimum value having clinical meaning.

In this way, the method of normalizing the subset of ECG data may be adapted according to the application of the search extraction algorithm, thereby increasing the accuracy of the search feature extraction.

In an embodiment, generating the search criterion comprises:
calculating a mean value for each of the plurality of normalized subsets of ECG data associated with the search feature;
defining a second threshold value for the search feature; and
generating the search criterion based on the mean value and the second threshold value.

In an embodiment, the search feature extraction algorithm comprises:
receiving a first input indicative of a research topic from an interface;
displaying at the interface a plurality of characteristic ECG features associated with the research topic and a predefined rule for each of the plurality of characteristic ECG features;
receiving a second input indicating a selected set of characteristic ECG features and rules;
applying the selected rules to the subsets of ECG data within the reference set of ECG data, wherein each subset of ECG data is associated with one of the selected characteristic ECG features; and
extracting one or more selected characteristic ECG features as search features when a percentage of a subset of ECG data associated with the one or more selected characteristic ECG features is consistent with the selected rule.

In this way, the search features may be extracted according to a known set of rules that are defined according to a given research topic, thereby simplifying the process for the user and generating search features that are only relevant to the given research project.

In an embodiment, the search feature extraction algorithm comprises:
obtaining a plurality of rules associated with a plurality of known characteristic ECG features, wherein each of the plurality of rules comprise a plurality of known clinical relationships;
applying the plurality of rules to the plurality of subsets of ECG data within the reference set of ECG data, wherein each of the subsets of ECG data is associated with one of the plurality of known characteristic ECG features; and
extracting one or more known characteristic ECG features as a search feature when a percentage of a subset of ECG data associated with the known feature characteristic ECG is consistent with the rule.

In this way, the search features may be extracted according to a known set of rules that are defined according to clinical knowledge. In other words, known parameters and relationships between data types may be used to define what is extracted as a search feature from the reference set of ECG data.

In an embodiment, the plurality of rules comprises one or more of:
a diagnosis;
a statement; and
a range of values.

In an embodiment, the method further comprises:
presenting a plurality of search features to a user; and
receiving a user input selecting one or more of the plurality of search features for generating the search criterion.

In this way, the user may select a desired search feature from a list of possible search features extracted by the algorithm.

According to examples in accordance with an aspect of the invention, there is provided a computer program comprising computer program code means which is adapted, when said computer program is run on a computer, to implement the methods described above.

According to examples in accordance with an aspect of the invention, there is provided a system for searching a database of ECG data, the system comprising:
a processor adapted to:
obtain a reference set of ECG data, wherein the reference set of ECG data comprises ECG data for a plurality of subjects comprising, for each of said subjects, data values for a plurality of characteristic ECG features;
group the reference set of ECG data into a plurality of subsets of ECG data, wherein each of the plurality of subsets of ECG data comprises data values for a respective one of the one of a plurality of characteristic ECG features;
apply a search feature extraction algorithm to the reference set of ECG data to extract at least one of said characteristic ECG features as a search feature wherein a search feature is a characteristic ECG feature which enables ECG data of interest to be identified within the database of ECG data;
generate a search criterion based on the search feature; and
search the database using the search criterion to obtain ECG data of interest.

In an embodiment, the processor is adapted, when applying the search feature extraction algorithm, to:
normalize each of the plurality of subsets of ECG data to generate a plurality of normalized subsets of ECG data;
calculate a variance for each of the plurality of normalized subsets of ECG data;
define one or more first threshold values;
compare the variance of each of the plurality of normalized subsets of ECG data with one of the one or more first threshold value; and
extract at least one feature as a search feature based on the comparison.

In an embodiment, the processor is adapted, when applying the search feature extraction algorithm, to:
receive a first input indicative of a research topic from an interface;
display at the interface a plurality of characteristic ECG features associated with the research topic and a predefined rule for each of the plurality of characteristic ECG features;
receive a second input indicating a selected set of characteristic ECG features and rules;
apply the selected rules to the subsets of ECG data within the reference set of ECG data, wherein each subset of ECG data is associated with one of the selected characteristic ECG features; and
extract one or more selected characteristic ECG features as search features when a percentage of a subset of ECG data associated with the one or more selected characteristic ECG feature is consistent with the selected rule.

In an embodiment, the processor is adapted, when applying the search feature extraction algorithm, to:
obtain a plurality of rules associated with a plurality of known characteristic ECG features, wherein each of the plurality of rules comprise a plurality of known clinical relationships;
apply the plurality of rules to the plurality of subsets of ECG data within the reference set of ECG data, wherein each of the subsets of ECG data is associated with one of the plurality of known characteristic ECG features; and
extract one or more known characteristic ECG features as a search feature when a percentage of a subset of ECG data associated with the known characteristic ECG feature is consistent with the rule.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a method for searching a database according to an aspect of the invention;
Figure 2 shows a first example of a search feature extraction algorithm;
Figure 3 shows a schematic representation of an example of a user interface according to an aspect of the invention;
Figure 4 shows a second example of a search feature extraction algorithm;
Figure 5 shows a schematic representation of an example of a user interface according to a further aspect of the invention;
Figure 6 shows a third example of a search feature extraction algorithm; and
Figure 7 shows a general computer architecture suitable for implementing the methods described herein.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for searching a database of ECG data. The method includes, obtaining a reference set of ECG data, wherein the reference set of ECG data comprises ECG data for a plurality of subjects comprising, for each of said subjects, data values for a plurality of characteristic ECG features, and grouping the reference set of ECG data into a plurality of subsets of ECG data, wherein each of the plurality of subsets of ECG data is associated with one of a plurality of characteristic ECG features. A search feature extraction algorithm is applied to the reference set of ECG data to extract at least one characteristic ECG feature as a search feature wherein a search feature is a characteristic ECG feature which enables ECG data of interest to be identified within the database of ECG data and a search criterion is generated based on the search feature. The database is then searched using the search criterion to obtain ECG data of interest.

The systems discussed herein may be implemented as part of any suitable processing system. The methods discussed herein may be performed using any suitable processing system.

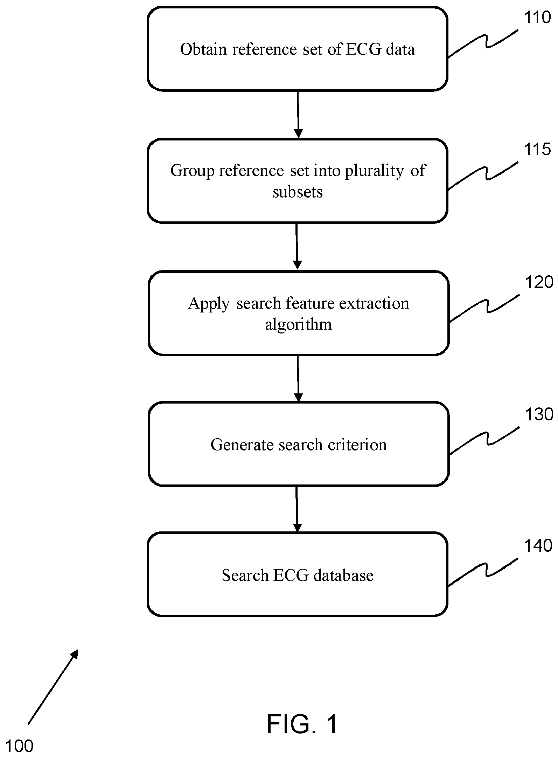

Figure 1 shows a method 100 for searching a database of ECG data.

In step 110, a reference set of ECG data is obtained, wherein the reference set of ECG data comprises ECG data for a plurality of subjects comprising, for each of said subjects, data values for a plurality of characteristic ECG features. The plurality of characteristic ECG features may include any features of an ECG wave, such as: P wave characteristics; Q notch characteristics; R peak characteristics; s notch characteristics; T wave characteristics; PR interval; QRS duration; the amplitude of the waves, notches or peaks; and the like.

For example, in a typical research program, a physician may collect several special cases that require further investigation as part of the research, which may then be treated as the reference set of data. For example, the reference set of data may include data relating to a plurality of subjects with ECG measurement values as the data, all having a certain disease or cardiac abnormality.

In step 115, the reference set of data is grouped into a plurality of subsets of, wherein each of the plurality of subsets of ECG data comprises data values for a respective one of the one of a plurality of characteristic ECG features.

By way of example, table 1 below provides an example of a reference set of data, wherein each row represents a different subject and each column represents a different subset of data corresponding to a characteristic ECG feature of the reference set of data. Put another way, all of the data points in each column of the table below share a common feature and form a subset of data when grouped together.

**Table 1: An example of a reference set of data comprising a plurality of features represented in the columns**

| Subject | Subject Statement | Ramp@I | Ramp@II | Ramp@III | Ramp@IV | Ramp@V |
|---|---|---|---|---|---|---|
| 1 | AGMUNK | 655 | 565 | 27 | 485 | 35 |
| 2 | SR RBBB | 413 | 278 | 55 | 322 | 199 |
| 3 | SR AMIAD | 521 | 377 | 77 | 480 | 0 |
| 4 | AGMUNK | 0 | 567 | 1530 | 0 | 191 |
| 5 | AGMUNK | 834 | 1211 | 950 | 594 | 0 |

In the example shown in table 1, Ramp@N means R wave amplitude value at Lead N in an ECG waveform, i.e. Ramp@I refers to R wave amplitude at Lead 1. Typically, there are 12 leads within an ECG wave, wherein the term lead refers to a line defined between two electrodes along which the signal is measured. Each piece of data in the table is taken from an ECG waveform obtained from a subject and calculated by way of an algorithm. The algorithm may extract a plurality of characteristic ECG features from the ECG waveform, such as amplitude of a wave or time interval between waves.

If the data includes a categorical value, for example a statement, such as a diagnosis or symptom, the user may be provided with a descriptive statistic indicating, for example, the data sharing the most frequent usage of the statement. For example, in table 1, AGMUNK may represent that the age and gender of the subject in that row is unknown. Further, SR may indicate that the Sinus rhythm is of interest, RBBB may indicate a right bundle-branch block and AMIAD may indicate an acute anterior infarction. Statements such as these may act as features in order to identify data of interest.

For example, the reference set of data may comprise one or more of: a numerical value representing a measurement obtained from one of the first plurality of subjects; a categorical value indicating a category of a measurement, and optionally wherein the categorical value comprises a statement relating to one of the first plurality of subjects.

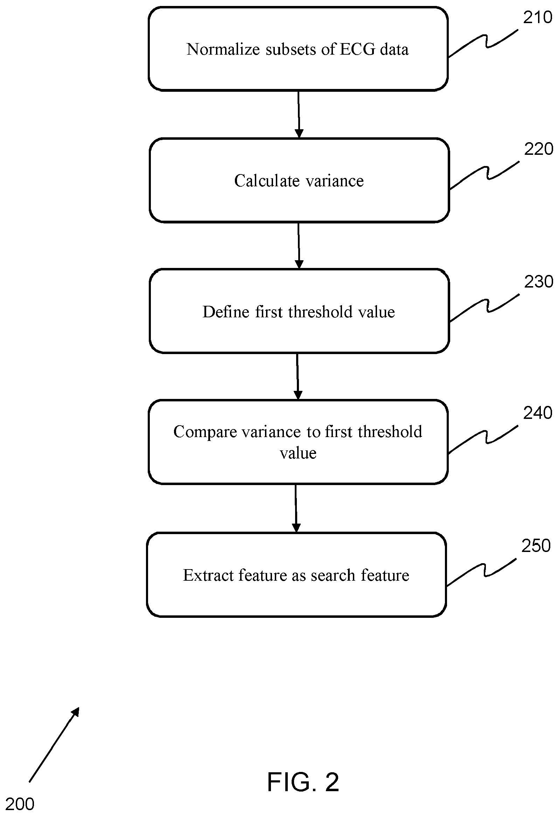

In step 120, a search feature extraction algorithm is applied to the reference set of ECG data to extract at least one of said characteristic ECG features as a search feature, wherein a search feature is a characteristic ECG feature which enables ECG data of interest to be identified within the database of ECG data. Several examples of search feature extraction algorithms are described further below with reference to figure 2 to 4.

In step 130, a search criterion is generated based on the search feature.

By way of example, the search criterion may comprise one or more of: equal to a mean value; not equal to a mean value; greater than a mean value; less than a mean value; and the like, wherein the mean value may be the mean of a subset of ECG data. The selection of the one or more search features may be performed automatically, for example, based on a known relationship between features or a detected anomaly in a descriptive statistic, or manually by way of a user input.

For example, the plurality of search features may be presented to a user by way of a user interface and the user may provide a user input selecting one or more of the plurality of search features for generating the search criterion.

In this way, the user may direct the generation of the search criterion in order to search for data of interest from the ECG database.

Further, a template expression of the search criterion may be displayed to the user by way of the user interface and a second user input may be received indicating an edit of the template expression to generate a search criterion based on the one or more search features and the second user input.

Put another way, before being finalized, the search criterion may be presented to a user for the purpose of editing the search criterion according to the desired search result.

In step 140, the database is searched using the search criterion to obtain ECG data of interest. The searched ECG data may then be utilized in any way in combination with, or independently of, the reference set of ECG data. The results of the search may be presented to the user by way of any suitable means of visualization, such as by way of the user interface.

Figure 2 shows an example 200 of a search feature extraction algorithm according to an aspect of the invention.

In the example 200 shown in Figure 2, the search feature extraction algorithm begins in step 210 by normalizing each of the plurality of subsets of ECG data to generate a plurality of normalized subsets of ECG data.

The normalization of the subsets of ECG data may be performed in a number of different ways according to the application of the searching method. For example, the subsets of ECG data based on a maximum and minimum value of the subset of ECG data. In other words, the subsets of ECG data may be normalized based on only the data contained within said subset of ECG data. Alternatively, the subsets of ECG data may be normalized based on a maximum and minimum value of the database of ECG data associated with the same characteristic ECG feature as the plurality of subsets of ECG data. Put another way, the subsets of ECG data may be normalized across the entire range of data contained within the ECG database for a given ECG feature. In a further example, the normalization of the subsets of ECG data may be performed based on a known maximum and minimum value having clinical meaning. In other words, the range of values used to normalize the subsets of ECG data may be predefined based on known clinical ranges for certain characteristic ECG features.

In step 220, a variance is calculated for each of the plurality of normalized subsets of ECG data.

The step of calculating descriptive statistics may then comprise, for each of the plurality of subsets of data within the reference set of data: for each subset of data comprising a numerical value, calculating at least one of a mean, a median, a standard deviation, a variance, a maximum and a minimum; or for the subset of data with a categorical value, calculating a percentage presence of each category within the reference set of data.

In the example, provided above in Table 1, the reference set of data comprises both numerical values representing measurements obtained from the reference plurality of subjects and categorical values in the statement column

**Table 2: An example of a reference set of data comprising a plurality of features represented in the columns and associated descriptive statistics**

| Subject | Subject Statement | Ramp@I | Ramp@II | Ramp@III | Ramp@IV | Ramp@V |
|---|---|---|---|---|---|---|
| 1 | AGMUNK | 655 | 565 | 27 | 485 | 35 |
| 2 | SR RBBB | 413 | 278 | 55 | 322 | 199 |
| 3 | SR AMIAD | 521 | 377 | 77 | 480 | 0 |
| 4 | AGMUNK | 0 | 567 | 1530 | 0 | 191 |
| 5 | AGMUNK | 834 | 1211 | 950 | 594 | 0 |
| Variance | | 78481 | 105809.4 | 372043.8 | 42902.6 | 8236.4 |

Table 2 shows the data of Table 1 with the addition of the variance of each column in the final row of the table as a descriptive statistic for each characteristic ECG feature. The variance may be replaced by any suitable descriptive statistic. Further, the categorical values, in the form of the statements in the statement column, may be used to generate descriptive statistics, such as a rate of occurrence of a given statement. By way of example, in Table 2, the statement AGMUNK occurs in 60% of subjects.

In step 230, one or more first threshold values are defined and in step 240, the variance of each of the plurality of normalized subsets of ECG data is compared with one of the one or more first threshold value. Defining the first threshold value may be performed in a number of ways according to the application of the search feature extraction algorithm.

For example, defining one or more first threshold values may comprise defining a first threshold value for a plurality of normalized subsets of ECG data, and wherein the variance of each of the plurality of subsets of ECG data relating to the same characteristic ECG features as the plurality of normalized subsets of ECG data are compared with said first threshold value. Put another way, a separate first threshold value may be set for each subset of ECG data according to the characteristic ECG feature of that subset.

Alternatively, defining one or more first threshold value may comprise defining a first threshold value for each of the plurality of subsets of ECG data, and wherein the variance of each of the plurality of subsets of ECG data are compared with the first threshold value defined for that subset of ECG data.

In step 250, at least one feature is extracted as a search feature based on the comparison.

In other words, extracting the search feature from the reference set of ECG data comprises determining a variance of a subset of ECG data and if the variance is less than or equal to a predetermined threshold, identifying the subset as a characteristic ECG feature to be used for extracting the search feature. Whereas, if the variance is above the predetermined threshold, the subset may be rejected.

Put another way, if the subset has a particularly high variance, i.e. a high degree of dissimilarity between the data, the subset of data may not result in a useful feature to be used for a search, and so would be rejected.

Alternatively, or in addition, determining the feature of the subset of data may include identifying a pattern in the subset of data, for example a portion of an ECG waveform, and determining a feature of the pattern to be use as a search feature.

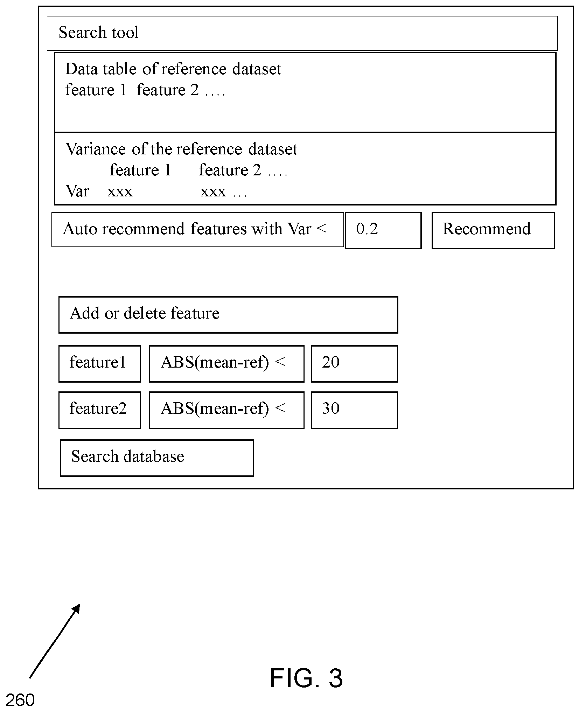

In practice, the search method and search feature extraction algorithm described above may proceed as follows. By way of example, Figure 3 shows a schematic representation of a user interface 260 implementing a worked example of the methods described above.

The user interface shows a data table of the data corresponding to the reference set of ECG data, wherein the data table comprises data divided into columns corresponding to a plurality of features (feature 1, feature 2 and the like).

From the user interface, which may be implemented as a screen, touchscreen and the like, the user may select a group of ECG data from an ECG data record to act as the reference set of ECG data. The group of data could be output from a previous search, or be imported directly. The group of data may have one or more unique similar characteristic ECG features to investigate further. For example, data from five subjects containing the same diagnostic result.

After the user has selected the data as the reference set of ECG data, the group of data is analyzed in order to obtain a search criterion.

A search feature extraction algorithm is then applied to the reference set of ECG data in order to extract search features. This may be performed by first normalizing the values of each data type and calculating the variance for each normalized data type. Then, by a predefined or manually set threshold, a certain subset of may be selected based on the calculated variance.

In the example shown in Figure 3, the user interface 360 comprises a table for displaying the variance (Var) for each column of data, thereby displaying a variance value for each characteristic ECG feature of the reference set of ECG data. Further, the user interface displays an automatic recommendation rule for recommending a subset of data to be selected by the user based on the calculated variance. In the example shown in Figure 3, features having a variance with an absolute value less than 0.2 are recommended to the user for selection.

After the selection of the subset of data, a series of values may be computed for each subset of data that can represent a feature of that data type, for example, an average value of a data type. The feature may then be used as a search feature to form the basis of a search criterion to search the whole ECG database. For example, the criteria may be *(Absolute value (searched* - *feature 1) < threshold) and (Absolute value (searched* - *feature2) < threshold).* After the search, the results may then be searched for data similar to the reference set of ECG data.

In the example shown in Figure 3, both feature 1 and feature 2 have been selected, either by the user or automatically, for example, because both features have a variance of less than 0.2. Features may be added or removed from the selection, for example, by the user selecting the Add or delete feature button. The selected characteristic ECG features may then form part of a condition formula for searching similar data from the ECG database. As shown in Figure 3, the condition formula states that for feature 1, the absolute value of the mean of the data obtained from the search of the ECG database must be less than 20 (feature1 ABS(mean-ref)< 20) and for feature 2, the absolute value of the mean of the data obtained from the search of the ECG database must be less than 30 (feature 2 ABS(mean-ref)< 30). The user may customize the parameters of the conditions accordingly. Further, multiple selected subsets of data will result in mapping the condition formula to multiple conditions and the logic relationship of these conditions may also be adjusted by the user, or automatically.

After the conditions are set, the condition function may be used as a search criterion and the user may initiate a search ECG database, for example by selecting the Search database button. An internal mechanism may then search the data from the ECG database that fulfils the conditions of the search criterion. Following the search, the obtained relevant data may be shown in a table on the user interface.

An alternative method to identify key features of the reference set of ECG data may utilize one or more of: similar ECG features; abnormalities in the ECG data; and a diagnosis associated with the ECG data in the group. For example, if the ECG data in the reference set of ECG data has similar statements or diagnoses, the characteristic ECG features related to such statements/diagnoses may be automatically recommended as search features. For example, where all of the ECG data in the reference set of ECG data shows myocardial infraction as a result, the ST segment of the ECG wave may be recommend as a search feature for similarity search.

Further, the ECG data may have similar features/patterns. For example, all of the ECG data in the reference set of ECG data may have an inverse T wave or a QRS notch. These features/patterns then be recommended for similarity search. In addition, ECG data having similar abnormalities, such as a high atrial rate, for example above 300/s and an ST segment in V1>0.2mV, the atrial rate and the ST segment may be recommended for a similarly search.

In a further alternative method, the search features may be manually selected. From a clinical point of view, certain subsets of data in the reference set of ECG data may have additional clinical meanings, or be clinically similar. For example, for a group of data including patients with the same symptoms or the same diseases, the user may directly select pieces of data and treat these as search features. Features may then be calculated from the selection of the user and used to search the ECG database.

More specifically, the user may directly select a certain number of columns, or subsets of data, and treat these columns as search features. After the selection of these columns, a series of values may be computed for each column that can represent a feature of the column, for example, a mean value of each column. The features of the selected columns may then be used as search features to search the ECG database. For example, the search criteria may include *(Absolute value (Measurement_value*_*of_all_data_feature1) < threshold_value)* and *(Absolute value (Measurement_value_of_all_data_feature2) < threshold value).*

The user may be provided with a user interface to customize how the features of the reference set of ECG data are calculated and/or used to build the search criterion.

For example, the user may customize one or more threshold values as criteria for each search feature. It may be possible to customize a threshold value for all the selections made by the user, or a set a values corresponding to different group statistics if the user selects groups of data.

Further, the user interface may include one or more checkboxes with research topics can be checked, and after checking, the measurements related to the research topics can be automatically selected. The user may also define one or more conditional relationships between the features, such as "AND" or "OR" or other logical calculators, in order to form the final combined search criteria.

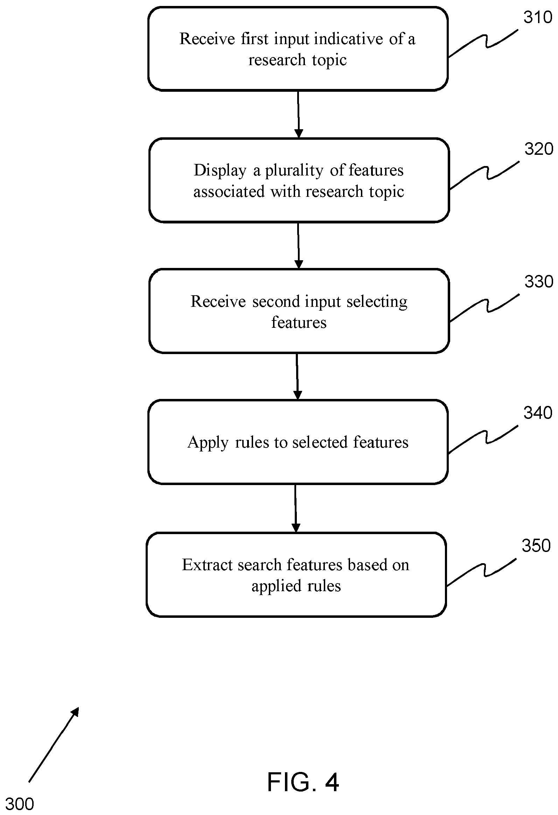

Figure 4 shows a further example 300 of a search feature extraction algorithm according to an aspect of the invention.

In the example 300 shown in Figure 4, the search feature extraction algorithm begins in step 310 by receiving a first input indicative of a research topic from an interface.

For example, a user may be presented with a list of possible research topics to select from or an interface by which to define a custom research project.

In step 320, a plurality of characteristic ECG features associated with the research topic and a predefined rule for each of the characteristic ECG plurality of features is displayed at the interface. The predefined rules define known or expected relationships between pieces of data. The relationships may be based on clinical knowledge, physical laws and the like.

In step 330, a second input indicating a selected set of characteristic ECG features and rules is received. Put another way, the user may select the rules, or relationships, that are of interest to the particular research topic.

In step 340, the selected rules are applied to the subsets of ECG data within the reference set of ECG data, wherein each subset of ECG data is associated with one of the selected characteristic ECG features and in step 350, one or more selected characteristic ECG features are extracted as search features when a percentage of a subset of ECG data associated with the one or more selected feature is consistent with the selected rule.

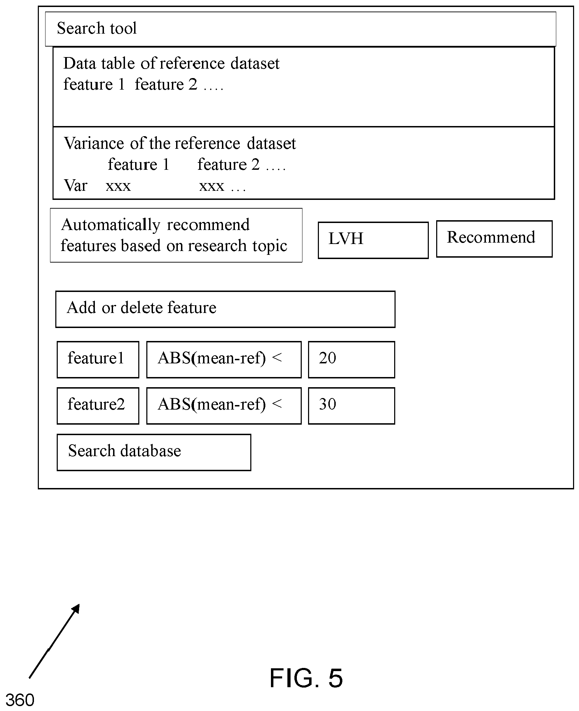

Figure 5 shows a schematic representation of a user interface 360 implementing a worked example of the methods described above, with reference to Figure 4.

As above in figure 3, the user interface 360 shows a data table of the data corresponding to the reference set of ECG data, wherein the data table comprises data divided into columns corresponding to a plurality of features (feature 1, feature 2 and the like). Further, the user interface 360 comprises a table for displaying the variance (Var) for each column of data, thereby displaying a variance value for each feature of the reference set of ECG data.

Further, the user interface displays an automatic recommendation rule for recommending a feature to be selected by the user based on the indicated research topic. In the example shown in Figure 3, features relating to left ventricle hypertrophy (LVH) are recommended to the user for selection.

In the example shown in Figure 3, both feature 1 and feature 2 have been selected, either by the user or automatically, for example, because both features relate to LVH. Features may be added or removed from the selection, for example, by the user selecting the Add or delete feature button. The selected features may then form part of a condition formula for searching similar data from the ECG database. As shown in Figure 5, the condition formula states that for feature 1, the absolute value of the mean of the data obtained from the search of the ECG database must be less than 20 (feature1 ABS(mean-ref)< 20) and for feature 2, the absolute value of the mean of the data obtained from the search of the ECG database must be less than 30 (feature 2 ABS(mean-ref)< 30). The user may customize the parameters of the conditions accordingly. Further, multiple selected subsets of data will result in mapping the condition formula to multiple conditions and the logic relationship of these conditions may also be adjusted by the user, or automatically.

After the conditions are set, the condition function may be used as a search criterion and the user may initiate a search ECG database, for example by selecting the Search database button. An internal mechanism may then search the data from the ECG database that fulfils the conditions of the search criterion. Following the search, the obtained relevant data may be shown in a table on the user interface.

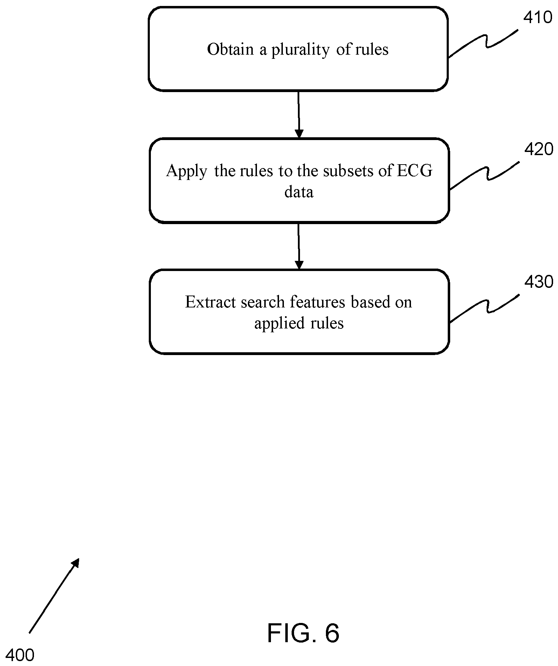

Figure 6 shows an example 400 of a search feature extraction algorithm according to an aspect of the invention.

In the example 400 shown in Figure 6, the search feature extraction algorithm begins in step 410 by obtaining a plurality of rules associated with a plurality of known characteristic ECG features, wherein each of the plurality of rules comprise a plurality of known clinical relationships. The plurality of rules may comprise one or more of: a diagnosis; a statement; and a range of values.

In other words, a rule list may be obtained that includes a plurality of rules which may be generated based on clinical knowledge, research papers, clinical guidelines and the like. The rules may, for example, include: diagnoses, which may be indicated by way of a statement such as LVH; ECG features that are abnormal or rare according to clinical knowledge, such as tamp@V1<0 (which corresponds to the amplitude of the T-wave at a given time), or heart rate >150; or a combination of several criteria with a known relationship, such as ston@V1 > 2000 (wherein ston represents the amplitude of the J point of an ECG wave, which is the junction between the QRS complex and the ST segment) AND tamp@V1 < -100.

In step 420, the plurality of rules are applied to the plurality of subsets of ECG data within the reference set of ECG data, wherein each of the subsets of ECG data is associated with one of the plurality of known characteristic ECG features.

In step 430, one or more known characteristic ECG features are extracted as a search feature when a percentage of a subset of ECG data associated with the known characteristic ECG feature is consistent with the rule. For example, if 80% or more of the subset of ECG data is consistent with the rule, then the characteristic ECG feature associated with the subset of data may be selected as a search feature. The percentage threshold for extracting a characteristic ECG feature as a search feature may be adjusted by the user or automatically.

**Table 3: An example of a rule list that may be applied to the reference set of ECG data**

| Name | Rule |
|---|---|
| High heart rate | Heart rate > 150 bpm |
| Anterior wall infarction, acute | Statement=AMIA |
| Sokolow-Lyon Criteria | S V1 + R V5 > 3.5 mV |
| Cornell Voltage Criteria (Women) | S V3 + R aVL > 2 mV and Sex=female |
| diagnosis of LVH | Statement = one of (LVH LVHR6 LVHS12 LVHEV LVHVAQ LVHRE LVHST LVHV LVHR56 LVHRSI LVH1 LVHCO LVHCOL TIALVH) |
| inversed T wave @ V1-V4 (all) | tamp@V1#leadMeasurements < -100 |
| | tamp@V2#leadMeasurements < -100 |
| | tamp@V3#leadMeasurements < -100 |
| | tamp@V4#leadMeasurements < -100 |

In the example shown in Table 3, the Sokolow-Lyon criteria is a set of criteria for diagnosing LVH, wherein S V1 represents the S wave amplitude at lead V1 and R V5 represents the R wave amplitude at lead V5. The Cornell Voltage criteria is a alternative set of criteria for diagnosing LVH, wherein R aVL represents the R wave amplitude at lead aVL.

The ECG system may have different statements related to LVH based on different criteria, as shown by the statements in the diagnosis of LVH row above. The statements are all given by algorithms related to LVH.

The methods described above may be used in combination with, or independently of, each other.

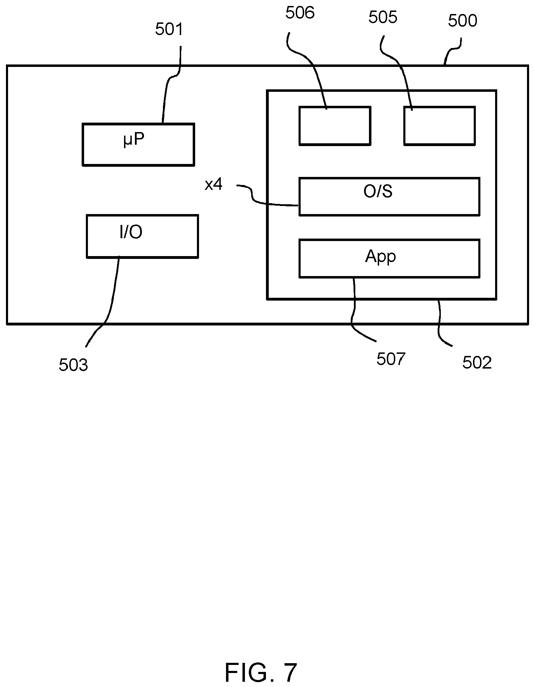

Figure 7 shows an example of a computer 500 for implementing the methods described above. The computer may be part of a cloud computing environment, a server or a standalone terminal.

The computer 500 includes, but is not limited to, PCs, workstations, laptops, PDAs, palm devices, servers, storages, and the like. Generally, in terms of hardware architecture, the computer 500 may include one or more processors 501, memory 502, and one or more I/O devices 503 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 501 is a hardware device for executing software that can be stored in the memory 502. The processor 501 can be virtually any custom made or commercially available processor, a central processing unit (CPU), a digital signal processor (DSP), or an auxiliary processor among several processors associated with the computer 500, and the processor 501 may be a semiconductor based microprocessor (in the form of a microchip) or a microprocessor.

The memory 502 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 502 may incorporate electronic, magnetic, optical, and/or other types of storage media. Note that the memory 502 can have a distributed architecture, where various components are situated remote from one another, but can be accessed by the processor 501.

The software in the memory 502 may include one or more separate programs, each of which comprises an ordered listing of executable instructions for implementing logical functions. The software in the memory 502 includes a suitable operating system (O/S) 504, compiler 505, source code 506, and one or more applications 507 in accordance with exemplary embodiments.

The application 507 comprises numerous functional components such as computational units, logic, functional units, processes, operations, virtual entities, and/or modules.

The operating system 504 controls the execution of computer programs, and provides scheduling, input-output control, file and data management, memory management, and communication control and related services.

Application 507 may be a source program, executable program (object code), script, or any other entity comprising a set of instructions to be performed. When a source program, then the program is usually translated via a compiler (such as the compiler 505), assembler, interpreter, or the like, which may or may not be included within the memory 502, so as to operate properly in connection with the operating system 504. Furthermore, the application 507 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 503 may include input devices such as, for example but not limited to, a mouse, keyboard, scanner, microphone, camera, etc. Furthermore, the I/O devices 503 may also include output devices, for example but not limited to a printer, display, etc. Finally, the I/O devices 503 may further include devices that communicate both inputs and outputs, for instance but not limited to, a network interface controller (NIC) or modulator/demodulator (for accessing remote devices, other files, devices, systems, or a network), a radio frequency (RF) or other transceiver, a telephonic interface, a bridge, a router, etc. The I/O devices 503 also include components for communicating over various networks, such as the Internet or intranet.

When the computer 500 is in operation, the processor 501 is configured to execute software stored within the memory 502, to communicate data to and from the memory 502, and to generally control operations of the computer 500 pursuant to the software. The application 507 and the operating system 504 are read, in whole or in part, by the processor 501, perhaps buffered within the processor 501, and then executed.

When the application 507 is implemented in software it should be noted that the application 507 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A computer implemented method (100) for searching a database of ECG data, the method comprising:
obtaining (110) a reference set of ECG data, wherein the reference set of ECG data comprises ECG data for a plurality of subjects comprising, for each of said subjects, data values for a plurality of characteristic ECG features;
grouping (115) the reference set of ECG data into a plurality of subsets of ECG data, wherein each of the plurality of subsets of ECG data comprises data values for a respective one of the one of a plurality of characteristic ECG features;
applying (120) a search feature extraction algorithm to the reference set of ECG data to extract at least one of said characteristic ECG features as a search feature wherein a search feature is a characteristic ECG feature which enables ECG data of interest to be identified within the database of ECG data;
generating (130) search criterion based on the search feature; and
searching (140) the database using the search criterion to obtain ECG data of interest.

2. A computer implemented method (100) as claimed in claim 1, wherein the search feature extraction algorithm comprises:
normalizing (210) each of the plurality of subsets of ECG data to generate a plurality of normalized subsets of ECG data;
calculating (220) a variance for each of the plurality of normalized subsets of ECG data;
defining (230) one or more first threshold values;
comparing (240) the variance of each of the plurality of normalized subsets of ECG data with one of the one or more first threshold value; and
extracting (250) at least one feature as a search feature based on the comparison.

3. A computer implemented method (100) as claimed in claim 2, wherein defining one or more first threshold values comprises defining a first threshold value for a plurality of normalized subsets of ECG data, and wherein the variance of each of the plurality of subsets of ECG data relating to the same characteristic ECG features as the plurality of normalized subsets of ECG data are compared with said first threshold value.

4. A computer implemented method (100) as claimed in claim 2, wherein defining one or more first threshold value comprises defining a first threshold value for each of the plurality of subsets of ECG data, and wherein the variance of each of the plurality of subsets of ECG data are compared with the first threshold value defined for that subset of ECG data.

5. A computer implemented method (100) as claimed in any of claims 2 to 4, wherein normalizing each of the plurality of subsets of ECG data comprises:
normalizing the subset of ECG data based on a maximum and minimum value of the subset of ECG data;
normalizing the subset of ECG data based on a maximum and minimum value of the database of ECG data associated with the same characteristic ECG feature as the plurality of subsets of ECG data; or
normalizing the subset of ECG data based on a known maximum and minimum value having clinical meaning.

6. A computer implemented method (100) as claimed in any of claims 2 to 5, wherein generating the search criterion comprises:
calculating a mean value for each of the plurality of normalized subsets of ECG data associated with the search feature;
defining a second threshold value for the search feature; and
generating the search criterion based on the mean value and the second threshold value.

7. A computer implemented method (100) as claims in claim 1, wherein the search feature extraction algorithm comprises:
receiving (310) a first input indicative of a research topic from an interface;
displaying (320) at the interface a plurality of characteristic ECG features associated with the research topic and a predefined rule for each of the plurality of characteristic ECG features;
receiving (330) a second input indicating a selected set of characteristic ECG features and rules;
applying (340) the selected rules to the subsets of ECG data within the reference set of ECG data, wherein each subset of ECG data is associated with one of the selected characteristic ECG features; and
extracting (350) one or more selected characteristic ECG features as search features when a percentage of a subset of ECG data associated with the one or more selected characteristic ECG features is consistent with the selected rule.

8. A computer implemented method (100) as claimed in claim 1, wherein the search feature extraction algorithm comprises:
obtaining (410) a plurality of rules associated with a plurality of known characteristic ECG features, wherein each of the plurality of rules comprise a plurality of known clinical relationships;
applying (420) the plurality of rules to the plurality of subsets of ECG data within the reference set of ECG data, wherein each of the subsets of ECG data is associated with one of the plurality of known characteristic ECG features; and
extracting (430) one or more known characteristic ECG features as a search feature when a percentage of a subset of ECG data associated with the known characteristic ECG feature is consistent with the rule.

9. A computer implemented method (100) as claimed in claim 8, wherein the plurality of rules comprises one or more of:
a diagnosis;
a statement; and
a range of values.

10. A computer implemented method (100) as claimed in any of claims 1 to 9, wherein the method further comprises:
presenting a plurality of search features to a user; and
receiving a user input selecting one or more of the plurality of search features for generating the search criterion.

11. A computer program comprising computer program code means which is adapted, when said computer program is run on a computer, to implement the method of any of claims 1 to 10.

12. A system for searching a database of ECG data, the system comprising:
a processor adapted to:
obtain a reference set of ECG data, wherein the reference set of ECG data comprises ECG data for a plurality of subjects comprising, for each of said subjects, data values for a plurality of characteristic ECG features;
group the reference set of ECG data into a plurality of subsets of ECG data, wherein each of the plurality of subsets of ECG data comprises data values for a respective one of the one of a plurality of characteristic ECG features;
apply a search feature extraction algorithm to the reference set of ECG data to extract at least one of said characteristic ECG features as a search feature wherein a search feature is a characteristic ECG feature which enables ECG data of interest to be identified within the database of ECG data;
generate a search criterion based on the search feature; and
search the database using the search criterion to obtain ECG data of interest.

13. A system as claimed in claim 12, wherein the processor is adapted, when applying the search feature extraction algorithm, to:
normalize each of the plurality of subsets of ECG data to generate a plurality of normalized subsets of ECG data;
calculate a variance for each of the plurality of normalized subsets of ECG data;
define one or more first threshold values;
compare the variance of each of the plurality of normalized subsets of ECG data with one of the one or more first threshold value; and
extract at least one feature as a search feature based on the comparison.

14. A system as claimed in claim 12, wherein the processor is adapted, when applying the search feature extraction algorithm, to:
receive a first input indicative of a research topic from an interface;
display at the interface a plurality of characteristic ECG features associated with the research topic and a predefined rule for each of the plurality of characteristic ECG features;
receive a second input indicating a selected set of characteristic ECG features and rules;
apply the selected rules to the subsets of ECG data within the reference set of ECG data, wherein each subset of ECG data is associated with one of the selected characteristic ECG features; and
extract one or more selected characteristic ECG features as search features when a percentage of a subset of ECG data associated with the one or more selected characteristic ECG feature is consistent with the selected rule.

15. A system as claimed in claim 12, wherein the processor is adapted, when applying the search feature extraction algorithm, to:
obtain a plurality of rules associated with a plurality of known characteristic ECG features, wherein each of the plurality of rules comprise a plurality of known clinical relationships;
apply the plurality of rules to the plurality of subsets of ECG data within the reference set of ECG data, wherein each of the subsets of ECG data is associated with one of the plurality of known characteristic ECG features; and
extract one or more known characteristic ECG features as a search feature when a percentage of a subset of ECG data associated with the known characteristic ECG feature is consistent with the rule.
